# EUROPEAN PATENT SPECIFICATION

(11) **EP 1 962 747 B1**
(45) Date of publication and mention of the grant of the patent: **16.03.2011**
(21) Application number: 06809985.2
(22) Date of filing: 20.09.2006
(51) Int. Cl.: A61F 5/02

(54) **ORTHOPEDIC SUPPORT OR ORTHESIS FOR THE SPINAL COLUMN**
ORTHOPÄDISCHE STÜTZE ODER ORTHESE FÜR DIE WIRBELSÄULE
SUPPORT ORTHOPEDIQUE OU ORTHESE POUR LA COLONNE VERTEBRALE

(30) Priority: 12.10.2005 IT VR20050125
(43) Date of publication of application: 03.09.2008
(73) Proprietor: F.G.P. Srl, 37062 Dossobuono (VR) (IT)
(72) Inventor: FERRIGOLO, Moreno, I-37062 Dossobuono (VR) (IT); TURRINI, Alberto, I-37062 CASTEL D1AZZANO (VR) (IT)
(74) Representative: Sandri, Sandro
(86) International application number: PCT/IT2006/000670
(87) International publication number: WO 2007/043079

(56) References cited:
- DE-A1- 3 232 638
- DE-U1- 29 720 475
- US-A- 2 828 737
- US-A- 3 945 376
- US-A1- 2004 133 138

## Description

### TECHNICAL FIELD

This invention concerns an orthopedic support or orthesis for the spinal column, which can be used to support and block the spinal column of subjects with articular problems of the back or suffering from osteoporosis.

More specifically, this invention refers to a support for the spinal column which, compared to known solutions, has the advantage of guaranteeing maximum comfort when worn and maximum safety in remaining adherent to the trunk even after flexion or other movements of the body.

The support according to the invention represents a jacket or brace-type orthesis for spinal joints or similar, and guarantees a greater or more or less perfect safety against the occurrence of accidental movements or unfastening of the brace, at the same time ensuring an excellent level of comfort.

This invention can be applied in the production sector of orthopedic jackets in general but also of prostheses and braces mainly used in conservative, post-traumatic, rehabilitation and post-operative therapy.

### BACKGROUND ART

It is known that with some diseases or some orthopedic type disorders of the spinal column or the trunk in persons subject for example to osteoporosis or other degenerative inflammatory disorders or following injury it is necessary to wear particular jacket-type braces or ortheses which guarantee a certain degree of support for the patient, absorbing the most intense stresses that the trunk is submitted to.

Useful in all types of backache in less acute phases of osteoporosis, by activating the muscles in the back, the orthesis favours straightening of the trunk, reducing the kyphois caused by osteoporosis.

Various types of jackets, braces or ortheses designed to support and contain the trunk are currently known and available. These are generally structures which rest against the spinal column and which are subject to some problems, in most cases due to the difficulty of attaching the brace to the trunk in a sufficiently stable way.

These devices mainly consist, in fact, of a rigid elongated frame, generally made from metal and shaped to adhere to the spinal column, the frame being fixed to the user's trunk by fastening means which are usually the strap or jacket type.

These strap fastening means consist of harnesses with ends attached to the rigid frame and which wrap around the trunk and are secured in place by appropriate adjustable type means of restraint.

The problem with this type of fastening is that the straps wrap around the user's body in a horizontal direction, passing from the back to the front, and since there are no restraining means to prevent vertical slipping, the orthesis can slip causing it to move from its correct position and lose adherence to the spinal column. This is also annoying for the user who often has to correct the position of the orthesis after even slight flexion movements of the body.

In other cases, and with the intention of preventing vertical slipping, the frame is instead encased in a special pouch positioned in the rear part of a bodice or of an elastic vest which is then worn by the subject.

In this case the bodice or vest can worsen the feeling of restriction and, because of their elasticity, they do not guarantee that the substantially rigid structure will be held firmly in place and perfectly adherent to the spinal column.

In all cases the main problem encountered with all the dorsal ortheses currently available is that they are unable to ensure the necessary stability of the rigid structure which must remain in contact with the spinal column, with all the consequent problems for the wearer.

Document US 2004/0133138 A1 discloses a spinal brace including a posterior support having a substantially rigid posterior splint, an anterior support having a substantially rigid anterior splint, and a pair of overlapping supports having substantially rigid splints which are releasably attached to the posterior support.

### DESCRIPTION OF THE INVENTION

This invention proposes to provide a dorsal orthesis for the correction and/or support of the spinal column, which can eliminate or at least reduce the problems described above.

In particular, the dorsal orthesis according to the invention proposes to resolve the problems caused by the possibility of slipping, particularly in a vertical direction along the trunk, of the traditional systems of restraint used so far.

The invention also proposes to provide a dorsal orthesis that can be easily produced in order to be economically advantageous as well as extremely efficient from the point of view of safety and stability.

This is achieved by means of an orthesis with the features described in the main claim.

The dependent claims describe advantageous embodiments of the invention.

The proposed aims are achieved, according to the invention, by a dorsal orthesis made by using special straps which are arranged to form a sort of harness starting from the orthesis itself covered by appropriately shaped covering.

The dorsal orthesis according to the invention thus has the particular feature of straps being distributed

over the trunk no longer in an exclusively horizontal direction, according to the traditional concepts currently used, but in an oblique direction and with securing lines that mainly prevent the orthesis from slipping upwards.

The straps are passed from the rear part of the trunk to the front using an abdominal connection plate positioned approximately at umbilical level, which guarantees that the structure remains firmly in position.

### DESCRIPTION OF THE DRAWINGS

Other features and advantages of the invention will become evident on reading the following description of one embodiment of the invention, provided as a non-binding example, with the help of the accompanying drawings in which:

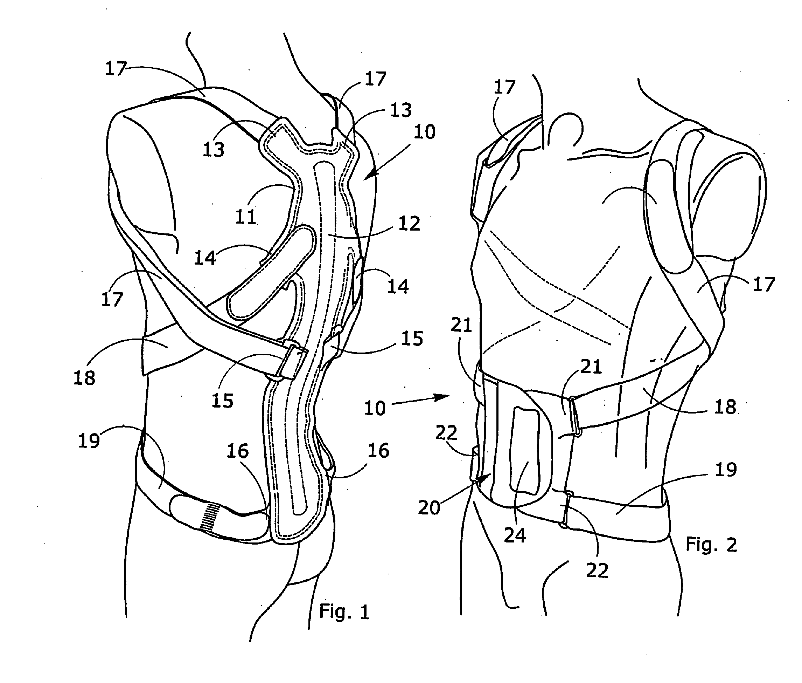

- figure 1 is a schematic and prospective view of the dorsal orthesis according to the invention seen from the rear three-quarters of the trunk;
- figure 2 is a schematic view of the front three-quarters;

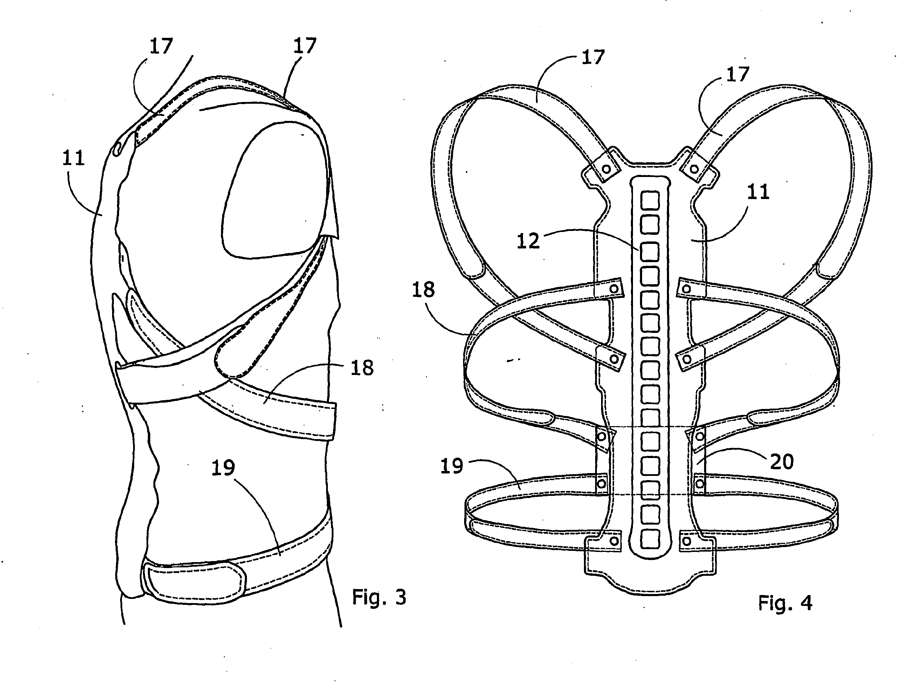

- figure 3 is a schematic side view of the orthesis according to the invention;
- figure 4 is a schematic rear view of the orthesis according to the invention;

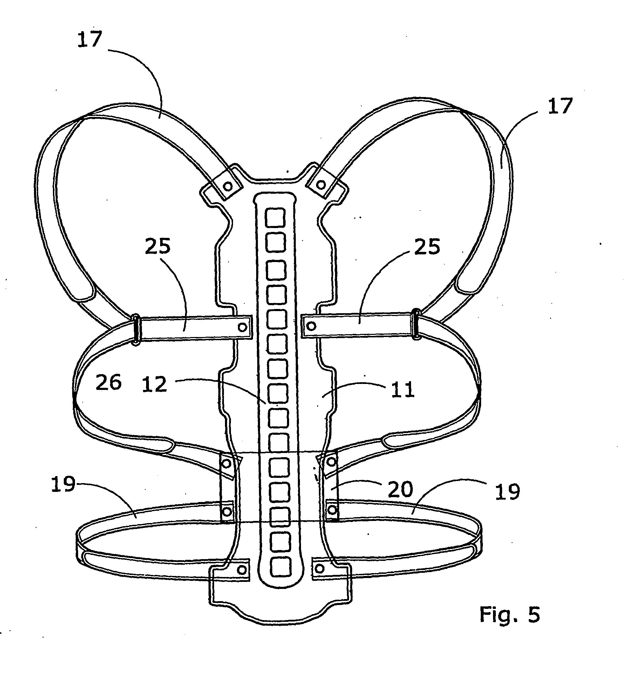

- figure 5 is a schematic rear view of the orthesis according to the invention in an alternative embodiment.

### DESCRIPTION OF ONE EMBODIMENT OF THE INVENTION

The orthesis according to the invention, mainly if not exclusively used in the orthopedic sector for the correct anatomical support and blocking of the spinal column, is indicated overall with the number 10 and substantially consists of a pocket-type element 11, made from soft cloth or velvet, with a vertically elongated shape and size designed to contain a rigid splint 12.

The rigid splint 12, made from metal or a material which is sufficiently rigid and resistant to adhere to and compress the spinal column in a corrective and anatomical way, is elongated and curved, according to the anatomical conformation of the trunk.

In specularly symmetrical points, the pocket element 11 presents attachment areas for straps to fasten the orthesis to the trunk of the wearer.

The strap attachment areas are defined by tabs which are indicated in the upper part by 13, in the upper central part by 14, in the lower central part by 15 and in the lower part by 16.

A number of straps lead from the attachment tabs 13, 14, 15 and 16. The straps are divided into three groups: the upper straps 17, which wrap around the shoulders, the middle straps 18 and the lower or lumbar straps 19 which respectively wrap around the sides of the body and the waist close to the buttocks.

The middle straps 18 and the lower straps 19 meet at the front in an abdominal connection plate 2, positioned in the umbilical area and with a substantially quadrilateral shape or other more appropriate conformation.

The abdominal plate 20 presents four fixing points for the same number of straps to be fastened.

The four fixing points on the abdominal plate 20 are indicated by 21 for the ends of the two middle straps 18 and by 22 for the ends of the lower or lumbar straps 19.

Each of the straps is fitted with opening and adjustment means 23, for example the Velcro type or other types of adjustment fastenings, while the abdominal plate 20 can be opened, also to make putting on the orthesis easier, by means of a flap 24 that can be secured with Velcro or another similar fastening system.

According to another embodiment, the orthesis can be made by joining the upper straps 17 and the middle straps 18, using ties 25 substantially positioned in the middle sector of the pocket 11 and equipped with rings 26 that act as a bridge between the upper 17 and lower 17' straps which meet at the abdominal plate 20.

As can be seen, the solution described above makes it possible to achieve all the proposed aims and in particular the harness in question for a dorsal orthesis allows the corrective splint 12 to remain in constant adherence against the spinal column, preventing any possibility of vertical slipping.

In fact, the middle straps 18 which are attached to the abdominal plate 20, resist the stress on the orthesis from above, preventing it from moving with respect to the wearer's spinal column.

The invention is described above with reference to a preferred embodiment. It is nevertheless clear that the invention is susceptible to numerous variations that lie within its scope, in the framework of technical equivalents.

## Claims

1. An orthopedic support (10) or dorsal orthesis which can be used to support and block the spinal column of subjects with articular back problems or suffering from osteoporosis and comprising at least one elongated rigid splint (12) with the anatomical shape of the spinal column, said splint (12) being contained inside a pocket (11), made from soft cloth or velvet, presenting attachment areas or tabs (13, 14, 15, 16) for straps (17, 18, 19) for fastening the orthesis to the trunk of a wearer, further comprising an abdominal connection plate (20) presenting tabs (21, 22) for straps (18, 19) **characterised by**
a) a first pair of upper straps (17) to be wrapped around the shoulders, each strap being fixed to a first upper attachment area (13) and to a lower tab (15) of said pocket (11);
b) a second pair of middle straps (18), each strap being respectively fixed to an upper tab (21) of said abdominal connection plate (20) and to a second upper attachment area (14) of said pocket (11);
c) said upper (17) and middle (18) straps crossing each other, in operation, in the side area of a user's body; and
d) a third pair of lower or lumbar straps (19), each strap being respectively fixed to a lower tab (22) of said abdominal connection plate (20) and to a lower attachment area (16) of said pocket (11).

2. An orthopedic support according to claim 1 **characterised in that** the abdominal connection plate (20) presents four fixing points (21, 22) for the same number of middle and lumbar straps (18, 19) to be fastened.

## Patentansprüche

1. Orthopädische Stütze oder Orthese für die Wirbelsäule, die eingesetzt werden kann, um die Wirbelsäule von Personen mit Rückengelenksproblemen oder von Personen, die an Osteoporose leiden, zu stützen und zu blockieren, mit zumindest einer länglichen steifen Schiene (12), die die anatomische Form der Wirbelsäule aufweist, wobei die Schiene (12) innerhalb einer Tasche (11) aufgenommen ist, die aus einem weichen Stoff oder Samt gemacht ist, und die Befestigungsbereiche oder -streifen (13, 14, 15, 16) für Gurte (17, 18, 19) aufweist, um die Orthese am Rumpf eines Trägers zu befestigen, und weiterhin mit einer Bauchverbindungsplatte (20), die Bereiche (21, 22) für Gurte (18, 19) aufweist, **gekennzeichnet durch**
a) ein erstes Paar von oberen Gurten (17), die um die Schultern geführt werden, wobei jeder Gurt an einem ersten oberen Anbringungsbereich (13) und an einem unteren Bereich (15) der Tasche (11) fixiert ist;
b) ein zweites Paar von mittleren Gurten (18), die jeweils an einem oberen Bereich (21) der Bauchverbindungsplatte (20) und an einem zweiten oberen Anbringungsbereich (14) der Tasche (11) fixiert sind;
c) wobei sich die oberen Streifen (17) und die mittleren Streifen (18) im seitlichen Bereich des Benutzers beim Tragen kreuzen; und
d) ein drittes Paar von unteren Gurten oder Lendengurten (19), wobei jeder Gurt jeweils an einem unteren Bereich (22) der Bauchverbindungsplatte (20) und an einem unteren Anbringungsbereich (16) der Tasche (11) fixiert ist.

2. Orthopädische Stütze nach Anspruch 1, **dadurch gekennzeichnet, dass** die Bauchverbindungsplatte (20) vier Befestigungspunkte (21, 22) für die gleiche Anzahl von mittleren Gurten und Lendengurten (18, 19) aufweist, die dort befestigt werden.

## Revendications

1. Support orthopédique (10) ou orthèse dorsale qui peut être utilisé pour supporter et bloquer la colonne vertébrale de sujets présentant des problèmes articulaires au niveau du dos ou souffrant d'ostéoporose ledit support comprenant au moins une éclisse rigide (12) de forme allongée en rapport avec la forme anatomique de la colonne vertébrale, ladite éclisse (12) étant contenue dans une poche (11), fabriquée à partir d'un tissu ou velours mou, présentant des zones de fixation ou attaches (13, 14, 15, 16) destinées à des sangles (17, 18, 19) pour fixer l'orthèse au tronc du porteur, comprenant en outre une plaque de connexion abdominale (20) présentant des attaches (21, 22) destinées à des sangles (18, 19), **caractérisé par**
a) une première paire de sangles supérieures (17) destinées à être enroulées autour des épaules, chaque sangle étant fixée à une première zone de fixation supérieure (13) et à une attache inférieure (15) de ladite poche (11) ;
b) une deuxième paire de sangles moyennes (18), chaque sangle étant respectivement fixée à une attache supérieure (21) de ladite plaque de connexion abdominale (20) et à une deuxième zone de fixation supérieure (14) de ladite poche (11) ;
c) lesdites sangles supérieures (17) et moyennes (18) se croisant, en utilisation, dans la zone latérale du corps d'un utilisateur ; et
d) une troisième paire de sangles inférieures ou lombaires (19), chaque sangle étant respectivement fixée à une attache inférieure (22) de ladite plaque de connexion abdominale (20) et à une zone de fixation intérieure (16) de ladite poche (11).

2. Support orthopédique selon la revendication 1, **caractérisé en ce que** la plaque de connexion abdominale (20) présente quatre points de fixation (21, 22) pour le même nombre de sangles moyennes et lombaires (18, 19) à fixer.
